# EUROPEAN PATENT APPLICATION

(11) **EP 4 108 238 A1**
(43) Date of publication of application: **28.12.2022**
(21) Application number: 21305849.8
(22) Date of filing: 21.06.2021
(51) Int. Cl.: A61K 31/138, A61K 31/501, A61K 31/5415, A61K 45/06, A61P 25/02, A61P 29/02

(54) **COMBINATION COMPRISING AT LEAST ONE SEROTONIN REUPTAKE INHIBITOR AND AT LEAST ONE STIMULATOR OF POTASSIUM-CHLORIDE COTRANSPORTER TYPE 2 AND ITS MEDICAL USE**

(71) Applicant: UNIVERSITE DE BORDEAUX, 33000 Bordeaux (FR); Centre national de la recherche scientifique, 75016 Paris (FR); Université Laval, Québec, Québec G1V 0A6 (CA)
(72) Inventor: FOSSAT, Pascal, 47000 AGEN (FR); ABY, Franck, 33000 BORDEAUX (FR); DE KONINCK, Yves, Québec, G1W 4P4 (CA); BOUALI-BENAZZOUZ, Rabia, 33600 PESSAC (FR)
(74) Representative: Novagraaf Technologies

(57) **Abstract**

The present invention relates to a combination comprising at least one serotonin reuptake inhibitor and at least one stimulator of potassium-chloride cotransporter type 2 (KCC2).

The present invention also relates to a pharmaceutical composition comprising at least one serotonin reuptake inhibitor and at least one stimulator of potassium-chloride cotransporter type 2 (KCC2), and to a kit of parts comprising at least one serotonin reuptake inhibitor and at least one stimulator of potassium-chloride cotransporter type 2 (KCC2), as a combined preparation for simultaneous, separate or sequential use.

## Description

### Technical field

The present invention refers to a combination of active agents, and its use in the treatment of painful sensations induced by peripheral neuropathy, by inflammation or by diabetes.

Therefore, the present invention has utility in the medical field.

### Background of the Invention

Pain is a warning system that protects organisms against real or potential tissue damages. This unpleasant feeling can be efficiently filtered in case of excessive pain thanks to the existence of an endogenous system that control pain transmission. This endogenous system is activated by diffuse noxious inhibitory control (DNIC) also called conditioned pain modulation (CPM) in human. Deficit in CPM is a good predictor of poor pain outcome after surgery, and growing evidences link abnormal CPM to chronic pain states in patients with postoperative, neuropathic or idiopathic pain. In this context, CPM paradigms are used to evaluate pain status. Alterations in CPM appear to result from an imbalance between excitatory and inhibitory descending pain pathways including monoaminergic nuclei (ie, serotonin and noradrenaline) of the brainstem that project to the dorsal horn of the spinal cord (DH). So far, underlying neural circuits remain largely unknown.

Therefore, the treatment of chronic neuropathic pain remains an important area of research. Indeed, current treatments have a very modest efficacy. Among these treatments, the first line is composed of tricyclic antidepressants (TCAs) and non-specific serotonin and noradrenaline reuptake inhibitors (SNRIs). Unfortunately, these molecules alone provide interesting but still very insufficient results. Indeed, the latest data from the International Association for the Study of Pain (IASP) NeuPSIG (International Neuropathic Pain Expert Group) show that the number of treatments needed to halve the pain in at least one patient is 3.6 for tricyclic antidepressants and 6.6 for SNRIs (NNT). These results were obtained after meta-analysis of 229 placebo-controlled studies.

On the other hand, these use of SNRIs leads to significant side effects and the number needed to stop treatment for adverse side effects (NNH) was 13.4 with tricyclics and 11.8 with SNRIs (data published by Finnnerup et al, in the Lancet neurology in 2016).

At the same time, studies with molecules specifically targeting serotonin (SSRIs) have little or no effect on the level of pain in neuropathic patients but have fewer adverse effects.

Thus, a need exists of alternative efficient and well-tolerated tools for treating chronic neuropathic pain.

### Description of the invention

The present invention fulfills these and other needs. Indeed, the Applicant has surprisingly found such an alternative solution for treating chronic pain.

The Applicant surprisingly has shown that an increase in the level of serotonin in the spinal cord is beneficial in neuropathic pain conditions if combined with a KCC2-type chloride transporter activator.

In particular, the Applicant has shown *in vivo,* in neuropathic mice, a superior effect of combination treatment with a serotonin reuptake inhibitor and a KCC2-type chloride transporter activator, compared to a treatment with a serotonin reuptake inhibitor or a KCC2-type chloride transporter activator alone. In particular, combination of fluoxetine, a serotonin reuptake inhibitor, injected intraperitoneally, with CLP290, a KCC2-type chloride transporter activator, by gavage (per os), resulted in an increased mechanical threshold response which is maintained at least 24 hours after injection. The combination fluoxetine/CLP resulted in a significant improvement in the mechanical response threshold which is significantly higher than fluoxetine and CLP alone.

Finally, the Applicant compared the efficacy of the combination of a serotonin reuptake inhibitor with a KCC2-type chloride transporter activator on a conditioned place preference (CPP) test. The Applicant showed that the combination increases the time spent by the animal in the treatment compartment, whereas this time is not modified by a combination serotonin reuptake inhibitor/solvent. This result shows that the combination serotonin reuptake inhibitor/KCC2-type chloride transporter activator brings an improvement of the general state of the animal, reinforcing the results obtained on the mechanical response thresholds.

Accordingly, in a first aspect, the present invention provides a combination comprising at least one serotonin reuptake inhibitor and at least one stimulator of potassium-chloride cotransporter type 2 (KCC2).

"Serotonin reuptake inhibitor" refers herein to any active agent increasing serotonin levels in the brain and the spinal cord. The serotonin reuptake inhibitor may be advantageously selected in the group consisting of serotonin specific reuptake inhibitors (SSRI), serotonin noradrenaline reuptake inhibitors (SNRI) and tricyclic antidepressants (TCA). Preferably, serotonin reuptake inhibitor may be a SSRI. In this case, the SSRI may be selected among fluoxetine, paroxetine, sertraline, citalopram, escitalopram oxalate, indalpine, zimelidine, dapoxetine and fluvoxamine maleate. Preferably, it may be fluoxetine. When serotonin reuptake inhibitor is a SNRI, it may be selected among desvenlafaxine, duloxetine, levomilnacipran and venlafaxine. When serotonin reuptake inhibitor is a TCA, it may be selected among amitriptyline, amoxapine, desipramine, doxepin, imipramine, nortriptyline, protriptyline and trimipramine.

"Stimulator of potassium-chloride cotransporter type 2 or KCC2" refers to any active agent capable of increasing KCC2 function and reducing the intracellular concentration of chloride ion. The stimulator of potassium-chloride cotransporter type 2 may be advantageously selected in the group consisting of molecules of the CLP family, which increase the number of chloride transporters of the KCC2 type at the membrane of spinal neurons, antipsychotic molecules of the piperazine phenothiazine. Molecules of the CLP family may be for example selected among CLP 290, CLP257 and CLP657. Antipsychotic molecules of the piperazine phenothiazine family may be prochlorperazine.

Advantageously, in the combination, the at least one serotonin reuptake inhibitor is fluoxetine, and the at least one stimulator of KCC2 is CLP 290.

"Combination" refers herein to a formulation wherein the at least one serotonin reuptake inhibitor and the at least one stimulator of KCC2 show synergistic effects in reducing painful sensations induced by peripheral neuropathy, by inflammation or by diabetes, either *in vitro* and *in vivo.* The term "synergy" or "synergistic" as used herein refers to a therapeutic combination which is more effective than the additive effects of the two single kinds of active agents, i.e. at least one serotonin reuptake inhibitor and at least one stimulator of KCC2. A determination of a synergistic interaction between at least one serotonin reuptake inhibitor and at least one stimulator of KCC2 may be based on the results obtained from the assays described herein. The results of these assays may be analyzed using the simplified up and down method for mechanical pain threshold assay and tracking system for conditioned placed preference assay. The combinations provided by this invention have been evaluated in several assay systems, and the data can be analyzed utilizing a standard program for quantifying synergism, additivism, and antagonism among agents.

According to the invention, a synergistic effect may be attained when the active agents are: (1) co-formulated and administered or delivered simultaneously in a combined, unit dosage formulation; or (2) delivered as separate formulations. When delivered in separate formulations, a synergistic effect may be attained when the compounds are administered or delivered simultaneous or sequentially, for example by different injections in separate syringes. In this case, the combination may be administered in two or more administrations, for example in consecutive administration in either order, wherein preferably there is a time period while both (or all) active agents simultaneously exert their biological activities. Whether by coadministration or by simultaneous, separate or sequential regimen, a therapeutically effective amount of each active ingredient is administered.

The combination of the invention may comprise a therapeutically effective amount of at least one serotonin reuptake inhibitor, and a therapeutically effective amount of the at least one stimulator of KCC2. The phrase "therapeutically effective amount" means an amount of a compound of the present invention that (i) treats the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In case of the treatment of painful sensations induced by peripheral neuropathy, by inflammation or by diabetes, the therapeutically effective amount of the above-mentioned active agents may reduce mechanical allodynia of about 52% 1h30 after injection (from 38% to 101%) and still about 36% 24hrs after injection (from 23 to 65%). As an example, a pharmaceutically effective amount of serotonin reuptake inhibitor administered per dose will be in the range of about 0.1-10mg/kg, depending on the route of administration, namely about 20mg (1 tablet) per day, with the typical initial range of compound used being 20 to 60 mg/day (i.e. for an adult of 70kg a doses of approx. 0.3mg/kg/day). Also as an example, a pharmaceutically effective amount of stimulator of KCC2 administered per dose will be in the range of about 10-100mg/kg, depending on the route of administration, namely about 10 to 100mg/kg of patient body weight per day, with the typical initial range of compound used being 10 to 100 mg/kg/day.

According to the invention, "at least one" refers to 1, or 2, or 3, or 4, or more of the cited active agent.

Another object of the invention relates to a pharmaceutical composition comprising at least one serotonin reuptake inhibitor and at least one stimulator of potassium-chloride cotransporter type 2 (KCC2). The at least one serotonin reuptake inhibitor and at least one stimulator of KCC2, as well as their therapeutically effective amount, are as defined above.

Serotonin reuptake inhibitor and stimulator of KCC2 may be formulated in the pharmaceutical composition in accordance with standard pharmaceutical practice for use in a therapeutic treatment in mammals including humans. Therefore, pharmaceutical compositions of the present invention include combinations of active agents as described above, and one or more pharmaceutically acceptable carrier, diluent, or excipient. The phrase "pharmaceutically acceptable" indicates that the substance or composition must be compatible chemically and/or toxicologically, with the other ingredients of the formulation, and the mammal being treated therewith. Suitable carriers, diluents and excipients are well known to those skilled in the art and include materials such as carbohydrates, waxes, water soluble and/or swellable polymers, hydrophilic or hydrophobic materials, gelatin, oils, solvents, water and the like. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, saline, cyclodextrin, ethanol, propylene glycol, polyethylene glycols (e.g., PEG 400, PEG 300), etc. and mixtures thereof. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents and flavoring agents.

The pharmaceutical compositions of the invention may be prepared using conventional dissolution and mixing procedures. For example, the bulk drug substance may be dissolved in a suitable solvent in the presence of one or more of the excipients described above.

The pharmaceutical compositions of the invention may be administered by any route appropriate to the condition to be treated, which includes oral and parenteral routes, the latter including subcutaneous, intramuscular, intravenous, intraarterial, intradermal, intrathecal, epidural, and infusion techniques. It will be appreciated that the preferred route may vary with for example the condition of the recipient. Where the composition is administered orally, it may be formulated as tablets, pills, hard or soft e.g., gelatin capsules, cachets, troches, lozenges, aqueous or oil suspensions, dispersible powders or granules, emulsions, syrups or elixirs each containing a predetermined amount of each active agent, with a pharmaceutically acceptable carrier, glidant, or excipient. Where the composition is administered parenterally, it may be formulated with a pharmaceutically acceptable parenteral vehicle or diluent, and in a unit dosage injectable form, as detailed below. For example, formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

Another object of the invention relates to a kit of parts comprising at least one serotonin reuptake inhibitor and at least one stimulator of potassium-chloride cotransporter type 2 (KCC2) as defined above, as a combined preparation for simultaneous, separate or sequential use. All the definitions given above are likely to apply mutatis mutandis to the kit of parts of the invention.

The "kit" according to the invention may be provided as an article of manufacture containing the active agents useful for the treatment of the diseases and disorders described above. The kit may comprise one container per active agent, or one container containing all the active agents. The kit may further comprise a label or package insert, on or associated with the container. The term "package insert" is used to refer to instructions customarily included in commercial packages of therapeutic products, that contain information about directions for the administration, the indications, usage, dosage, administration, contraindications and/or warnings concerning the use of such therapeutic products. Especially, if the kit comprises a first formulation comprising one serotonin reuptake inhibitor and a second formulation comprising one stimulator of KCC2, the kit may further comprise directions for the simultaneous, sequential or separate administration of the two formulations to a patient in need thereof.

Another object of the invention relates to the combination as defined above, the pharmaceutical composition as defined above, or the kit of parts as defined above, for medical use.

More particularly, the medical use may be the treatment of painful sensations induced by peripheral neuropathy, by inflammation or by diabetes.

This invention is further illustrated by the following examples with regard to the annexed drawings that should not be construed as limiting.

### Brief description of the figures

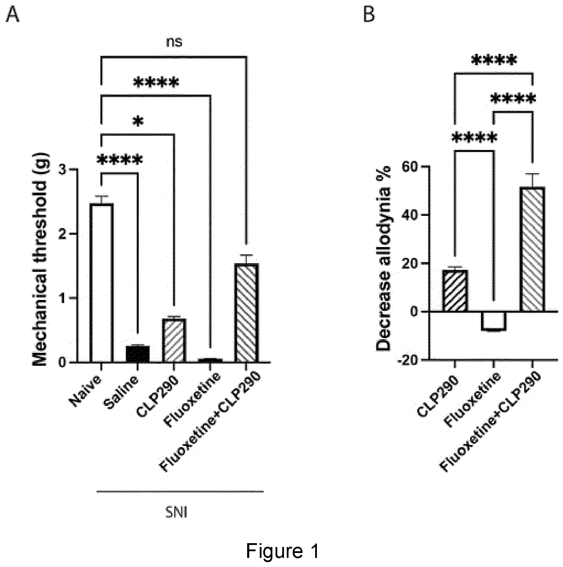

- Figure 1: A) mechanical threshold (in g) evaluated in a naive group of mice, in SNI groups, in a group of SNI injected intraperitoneally with fluoxetine 10mg/kg, in a group of SNI injected by gavage with CLP290 100mg/kg and a group of SNI injected with both fluoxetine and CLP290. Mechanical evaluation is performed 1h30 with drug injection, as explained in Example 1. B) decreased mechanical allodynia (in %) after treatments with fluoxetine, CLP290 and association of both molecules.

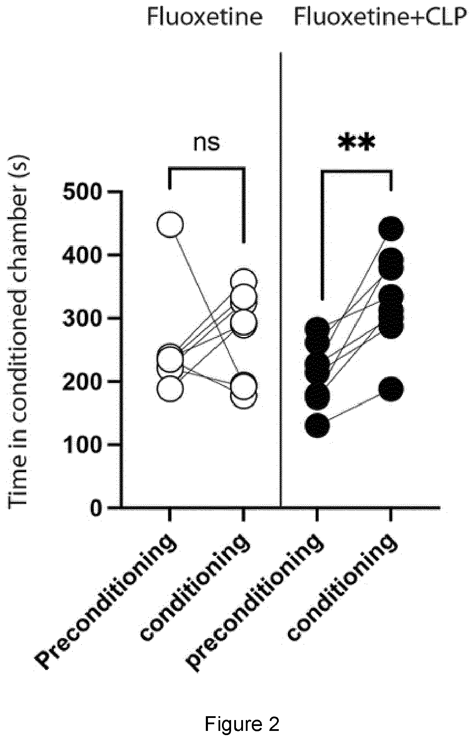

- Figure 2: represents the time in conditioned chamber (in s) neuropathic mice treated with either fluoxetine alone (white circles) or combination of fluoxetine and CLP290 (black circles), during preconditioning and conditioning, as explained in Example 2.

### Examples

### Example 1: Reversal of mechanical allodynia by association of KCC2 enhancer, CLP290 and serotonin specific reuptake inhibitor, fluoxetine

Mice are divided in 5 experimental groups. 1) Naive group that only receive mechanical threshold evaluation (g). 2) SNI (spinal nerve injury) groups that receive a unilateral ligature and section of 2 of the three main branches of the right sciatic nerve. 14 days after surgical procedure, mechanical threshold (g) is evaluated in a group of SNI, a group of SNI injected intraperitoneally with fluoxetine 10mg/kg, a group of SNI injected by gavage with CLP290 100mg/kg and a group of SNI injected with both fluoxetine and CLP290.

Mechanical evaluation is performed 1h30 with drug injection.

Threshold for mechanical withdrawal is assessed by means of Von Frey filaments. Values in grams (g) is the force needed to induce a paw withdrawal (see Figure 1A)). SNI procedures induce a decrease in mechanical threshold showing a mechanical allodynia. Treatment with CLP290 and fluoxetine relieved this allodynia as mechanical threshold is not significantly different with naive animals.

Figure 1B) shows the percentage of decreased mechanical allodynia after treatments with fluoxetine, CLP290 and association of both molecules. Association of CLP290 with fluoxetine reduced the amplitude of mechanical allodynia by 51.8 %.

### Example 2: Conditioned place preference (CPP) test

A spinal nerve injury is performed as in example 1.

14 days after neuropathic mice are free to explore during 10 minutes, a 2-chamber arena separated by a narrow corridor. A camera placed above the device allows tracking of mice movements. This exploration is considered the pre-conditioning condition in figure 2. 1H30 after injection of either fluoxetine alone or combination of fluoxetine and CLP290, mice are restricted to one chamber during 10 minutes. 2 days after mice are put again and free to explore the entire arena. If the treatment improved mice "well-being", mice will spend more time in the chamber associated with the treatment. This is what happened for the combination CLP290 and fluoxetine but not for fluoxetine alone, as shown in Figure 2. As control experiment, the same procedure is performed in neuropathic mice with fluoxetine and CLP290 vehicle.

Thus, association of both fluoxetine and CLP290 increases the time spent by neuropathic mice in the conditioned chamber, but not with fluoxetine alone. This preference for the compartment associated with the molecular formula of the invention is related to an increase "well-being" associated to this formula.

## Claims

1. A combination comprising at least one serotonin reuptake inhibitor and at least one stimulator of potassium-chloride cotransporter type 2 (KCC2).

2. A combination according to claim 1, wherein said serotonin reuptake inhibitor is selected in the group consisting of serotonin specific reuptake inhibitors (SSRI), serotonin noradrenaline reuptake inhibitors (SNRI) and tricyclic antidepressants (TCA).

3. A combination according to claim 2, wherein said SSRI is fluoxetine.

4. A combination according to anyone of claims 1 to 3, wherein said stimulator of KCC2 is selected in the group consisting of molecules of the CLP family and antipsychotic molecules of the piperazine phenothiazine family.

5. A combination according to claim 4, wherein said molecules of the CLP family is chosen among CLP 290, CLP657 and CLP257.

6. A combination according to claim 4, wherein said antipsychotic molecules of the piperazine phenothiazine family is prochlorperazine.

7. A combination according to anyone of the preceding claims, wherein said at least one serotonin reuptake inhibitor is fluoxetine, and said at least one KCC2 is CLP 290.

8. A pharmaceutical composition comprising at least one serotonin reuptake inhibitor and at least one stimulator of potassium-chloride cotransporter type 2 (KCC2) as defined in claims 1 to 7.

9. A kit of parts comprising at least one serotonin reuptake inhibitor and at least one stimulator of potassium-chloride cotransporter type 2 (KCC2) as defined in claims 1 to 7, as a combined preparation for simultaneous, separate or sequential use.

10. The combination according to any one of claims 1 to 7, the pharmaceutical composition according to claim 8, or the kit of parts according to claim 9 for medical use.

11. The combination according to any one of claims 1 to 7, the pharmaceutical composition according to claim 8, or the kit of parts according to claim 9 for use in the treatment of painful sensations induced by peripheral neuropathy, by inflammation or by diabetes.

12. The combination, the pharmaceutical composition or the kit of parts for use according to claim 11, wherein said at least one serotonin reuptake inhibitor and said at least one stimulator of KCC2 are administered orally.
